# EUROPEAN PATENT APPLICATION

(11) **EP 4 647 103 A1**
(43) Date of publication of application: **12.11.2025**
(21) Application number: 24832347.9
(22) Date of filing: 17.06.2024
(51) Int. Cl.: A61M 25/01, A61M 5/42, A61M 25/06, A61M 5/46, A61B 5/15, A61B 5/00, A61B 90/00

(54) **INVASIVE INSTRUMENT GUIDE DEVICE**

(30) Priority: 26.06.2023 KR 20230081862
(71) Applicant: Airs Medical Inc., Seoul 08788 (KR)
(72) Inventor: KIM, Changkyun, Siheung-si Gyeonggi-do 14991 (KR)
(74) Representative: Isarpatent
(86) International application number: PCT/KR2024/008315
(87) International publication number: WO 2025/005567

(57) **Abstract**

According to an embodiment of the present disclosure, there is provided an invasive instrument guide device including: a first adapter part configured to be coupled with a probe that radiates an ultrasound beam toward a target location, and to fix the location of the probe; a second adapter part configured to be coupled with an invasive instrument, and to be moved together with the invasive instrument; a guide part configured to be connected to the second adapter part, and to guide the second adapter part through its movement along the longitudinal direction of the invasive instrument; and a limitation part including a stopper configured to be arranged on the movement path of the second adapter part and limit the movable distance of the second adapter part; wherein the limitation part is controlled such that the movable distance of the second adapter part is changed.

## Description

### Technical Field

The present disclosure relates to an invasive instrument guide device. More specifically, the present disclosure relates to an invasive instrument guide device capable of guiding needles having different lengths through their accurate insertion up to a target location.

### Background Art

In general, invasive procedures for living bodies are performed for purposes such as blood collection, catheter sheath insertion, drug injection, etc.

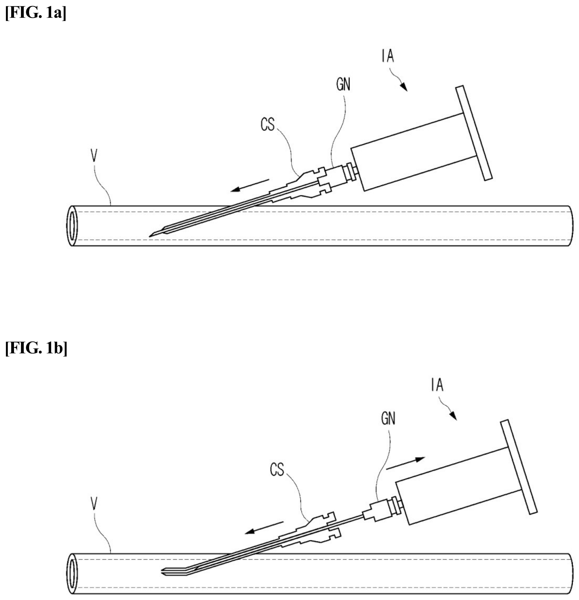

FIG. 1 shows diagrams illustrating an example of an invasive procedure for a living body, which show an intravenous (IV) injection method using a catheter sheath CS and a guide needle GN.

First, an invasive instrument IA having a form in which a guide needle GN and a catheter sheath CS overlap each other is stuck into the skin and inserted into a vein V (see FIG. 1(a)). Once the invasive instrument IA has been inserted into the vein V, the catheter sheath CS is pushed further into the vein V while the guide needle GN is pulled out rearward (see FIG. 1(b)).

Invasive procedures for living bodies such as the above-described intravenous injection require the precise insertion of needles into blood vessels of invasion targets, so that it is important to locate blood vessels of the invasion targets. When blood vessels of invasion targets are not clearly visible, it is difficult to locate blood vessels, making invasive procedures difficult.

To overcome this problem, there are being developed invasive instrument guide devices that provide guidance by using ultrasounds to accurately locate blood vessels during invasive procedures for living bodies.

These devices may visually show blood vessels and invasive instruments by emitting ultrasounds along the movement paths of the invasive instruments. Accordingly, a user who wants to insert the needle of an invasive instrument into his or her own or another person's living body may insert the needle up to an appropriate location while directly viewing an ultrasound image generated by a probe.

However, even when these devices are used, the depth at which the needle is inserted will inevitably vary depending on the user's skill level. Furthermore, there is always a possibility that posterior wall puncture may occur in the case of an unskilled user or by mistake.

Meanwhile, the invasive instrument allows various needle thicknesses to be selected as needed, in which case the length of the needle also varies depending on the thickness of the needle. For example, an 18G (Gauge) needle has a thicker and longer shape than a 24G needle.

Therefore, always guiding needles having various lengths through their accurate insertion into a target location also needs to be taken into consideration in the development of invasive instrument guide devices.

### Disclosure

### Technical Problem

The present disclosure has been conceived in response to the above-described background art, and an object of one embodiment of the present disclosure is to provide an invasive instrument guide device capable of guiding an invasive instrument through its movement path so that a needle may be accurately inserted up to a target location.

Another object of one embodiment of the present disclosure is to provide an invasive instrument guide device capable of limiting the movable distance of an invasive instrument so that even when the length of a needle is changed, the needle may always be accurately inserted up to the same target location.

Another object of one embodiment of the present disclosure is to provide an invasive instrument guide device capable of preventing a portion of a needle from being covered by the invasive instrument guide device, thereby allowing the overall length of even a needle having a short length to be inserted up to a target position.

Still another object of one embodiment of the present disclosure is to provide an invasive instrument guide device capable of preventing a needle from coming into contact with one component of the invasive instrument guide device, thereby allowing an invasive procedure for a living body to be always performed hygienically.

However, the objects to be achieved in the present disclosure are not limited to the object mentioned above, and other objects not mentioned may be clearly understood based on the following description.

### Technical Solution

In order to accomplish the above-described objects, there is disclosed an invasive instrument guide device according to one embodiment of the present disclosure.

According to an embodiment of the present disclosure, there is provided an invasive instrument guide device including: a first adapter part configured to be coupled with a probe that radiates an ultrasound beam toward a target location, and to fix the location of the probe; a second adapter part configured to be coupled with an invasive instrument, and to be moved together with the invasive instrument; a guide part configured to be connected to the second adapter part, and to guide the second adapter part through its movement along the longitudinal direction of the invasive instrument; and a limitation part including a stopper configured to be arranged on the movement path of the second adapter part and limit the movable distance of the second adapter part; wherein the limitation part is controlled such that the movable distance of the second adapter part is changed.

Alternatively, the first adapter part may include: a probe fixation portion configured such that the probe is coupled and fixed thereto; and an adapter body configured to be connected to the probe fixation portion, and to accommodate the guide part and the limitation part in the internal space thereof.

Alternatively, the second adapter part may include: an invasive instrument fixation portion configured such that the invasive instrument is coupled and fixed thereto; and a movement portion configured such that one side thereof is connected to the invasive instrument fixation portion and the other side thereof is connected to the guide part, and such that it is slid along the longitudinal direction.

Alternatively, the invasive instrument may include: a needle part configured to invade a living body; and a coupling part configured to be connected to the needle part, and to be coupled to the invasive instrument fixation portion.

Alternatively, the limitation part may further include: a limitation part body; and a rotary knob configured to be connected to one side of the limitation part body in the longitudinal direction thereof, and to be rotated by an external force; and the stopper may be connected to one side of the limitation part body in the radial direction thereof, and may be arranged on the movement path of the second adapter part.

Alternatively, the limitation part body may have a cylindrical shape, and may be provided with a spiral stopper movement groove formed along the outer circumferential surface thereof.

Alternatively, the stopper may be configured such that one side thereof is connected to the stopper movement groove and the other side thereof is connected to the guide part.

Alternatively, a plurality of numbers each indicating the gauge specification or length of a needle included in the invasive instrument may be marked on the rotary knob.

Alternatively, the limitation part body may be rotated in conjunction with the rotary knob when the rotary knob is rotated.

Alternatively, the stopper may be moved forward or rearward along the longitudinal direction when the rotary knob is rotated.

### Advantageous Effects

The invasive instrument guide device according to one embodiment of the present disclosure includes the second adapter part configured such that the invasive instrument is coupled thereto and the guide part configured to guide the second adapter part through its sliding along the longitudinal direction of the invasive instrument. Accordingly, there is an effect in that a needle may be accurately inserted up to a target location.

Furthermore, the invasive instrument guide device according to one embodiment of the present disclosure is configured such that the movable distance of the second adapter part is variable. Accordingly, there is an effect in that even when the length of the needle is changed, the needle may always be accurately inserted up to the same target location.

Furthermore, the invasive instrument guide device according to one embodiment of the present disclosure is configured such that the coupling part of the invasive instrument is fixed to the second adapter part and the needle part that directly invades a living body is exposed out of the invasive instrument guide device. Accordingly, there is an effect in that a portion of a needle is not covered by the invasive instrument guide device, so that the overall length of even a needle having a short length can be inserted up to a target position.

Moreover, the invasive instrument guide device according to one embodiment of the present disclosure is configured such that the coupling part of the invasive instrument is fixed to the second adapter part and the needle part that directly invades a living body is exposed out of the invasive instrument guide device. Accordingly, there is an effect in that the needle does not come into contact with one component of the invasive instrument guide device, so that an invasive procedure for a living body may always be performed hygienically.

The effects of the present disclosure are not limited to the effects mentioned above, and other effects not mentioned may be clearly understood by those skilled in the art from the description of the claims.

### Description of Drawings

FIG. 1 shows diagrams illustrating an example of an invasive procedure for a living body, which show an intravenous injection method using a catheter sheath and a guide needle;
FIG. 2 shows diagrams illustrating another example of an invasive procedure for a living body, which show a method using a blood collection needle and a blood container;
FIG. 3 is a diagram showing an invasive instrument guide device according to one embodiment of the present disclosure;
FIG. 4 is a diagram showing the rotary knob of an invasive instrument guide device according to one embodiment of the present disclosure;
FIG. 5 is a diagram illustrating the movement of a stopper in an invasive instrument guide device according to one embodiment of the present disclosure;
FIG. 6 shows diagrams illustrating an example in which an invasive instrument having a first specification is guided through its insertion by an invasive instrument guide device according to one embodiment of the present disclosure; and
FIG. 7 shows diagrams illustrating an example in which an invasive instrument having a second specification is guided through its insertion by an invasive instrument guide device according to one embodiment of the present disclosure.

### Mode for Invention

Embodiments of the present disclosure will be described in detail below with reference to the accompanying drawings so that those having ordinary skill in the art of the present disclosure (hereinafter, those skilled in the art) can easily implement the present disclosure. The embodiments presented in the present disclosure are provided to enable those skilled in the art to use or practice the content of the present disclosure. Accordingly, various modifications to embodiments of the present disclosure will be apparent to those skilled in the art. That is, the present disclosure may be implemented in various different forms and is not limited to the following embodiments.

The same or similar reference numerals denote the same or similar components throughout the specification of the present disclosure. Additionally, in order to clearly describe the present disclosure, reference numerals for parts that are not related to the description of the present disclosure may be omitted in the drawings.

The term "or" used herein is intended not to mean an exclusive "or" but to mean an inclusive "or." That is, unless otherwise specified herein or the meaning is not clear from the context, the clause "X uses A or B" should be understood to mean one of the natural inclusive substitutions. For example, unless otherwise specified herein or the meaning is not clear from the context, the clause "X uses A or B" may be interpreted as any one of a case where X uses A, a case where X uses B, and a case where X uses both A and B.

The term "at least one of A or B" used herein should be interpreted to refer to all combinations of A, B, and A and B.

The term "and/or" used herein should be understood to refer to and include all possible combinations of one or more of listed related concepts.

The terms "include" and/or "including" used herein should be understood to mean that specific features and/or components are present. However, the terms "include" and/or "including" should be understood as not excluding the presence or addition of one or more other features, one or more other components, and/or combinations thereof.

Unless otherwise specified herein or unless the context clearly indicates a singular form, the singular form should generally be construed to include "one or more."

The term "N-th (N is a natural number)" used herein can be understood as an expression used to distinguish the components of the present disclosure according to a predetermined criterion such as a functional perspective, a structural perspective, or the convenience of description. For example, in the present disclosure, components performing different functional roles may be distinguished as a first component or a second component. However, components that are substantially the same within the technical spirit of the present disclosure but should be distinguished for the convenience of description may also be distinguished as a first component or a second component.

Meanwhile, the term "module" or "unit" used herein may be understood as a term referring to an independent functional unit processing computing resources, such as a computer-related entity, firmware, software or part thereof, hardware or part thereof, or a combination of software and hardware. In this case, the "module" or "unit" may be a unit composed of a single component, or may be a unit expressed as a combination or set of multiple components. For example, in the narrow sense, the term "module" or "unit" may refer to a hardware component or set of components of a computing device, an application program performing a specific function of software, a procedure implemented through the execution of software, a set of instructions for the execution of a program, or the like. Additionally, in the broad sense, the term "module" or "unit" may refer to a computing device itself constituting part of a system, an application running on the computing device, or the like. However, the above-described concepts are only examples, and the concept of "module" or "unit" may be defined in various manners within a range understandable to those skilled in the art based on the content of the present disclosure.

The term "connected" used therein should be interpreted to include not only the case where components are "directly connected" to each other, but also the case where another component is "present" between components and the case where components are "electrically connected" with another component disposed therebetween.

The foregoing descriptions of the terms are intended to help understanding of the present disclosure. Accordingly, it should be noted that unless the above-described terms are explicitly described as limiting the content of the present disclosure, the terms in the content of the present disclosure are not used in the sense of limiting the technical spirit of the present disclosure.

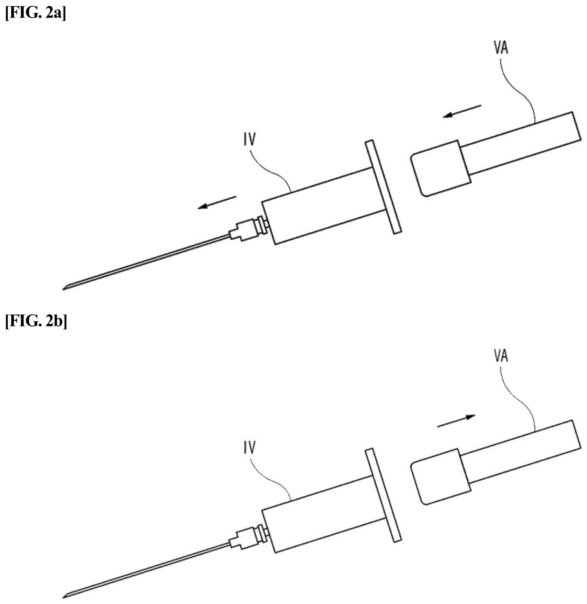

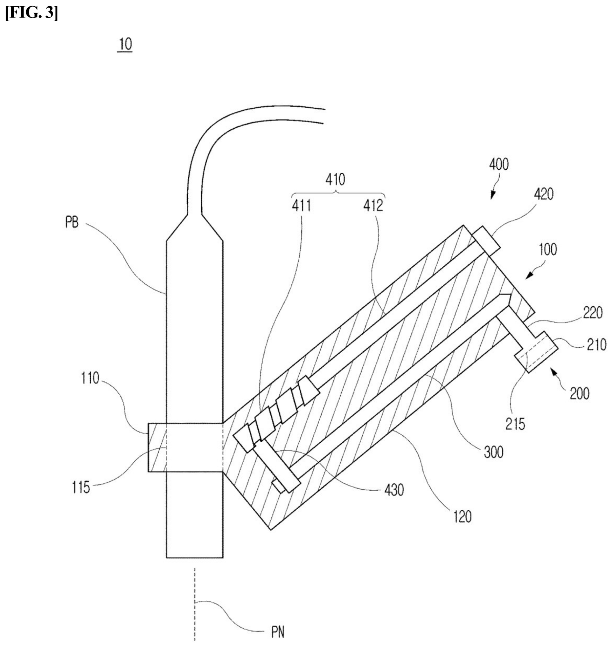

FIG. 2 shows diagrams FIG. 2 illustrating another example of an invasive procedure for a living body, which show a method using a blood collection needle and a blood container. FIG. 3 is a diagram showing an invasive instrument guide device according to one embodiment of the present disclosure.

First, referring to FIG. 3, an invasive instrument guide device 10 according to one embodiment of the present disclosure may include a first adapter part 100, a second adapter part 200, a guide part 300, and a limitation part 400.

The first adapter part 100 may be coupled with a probe PB that radiates an ultrasound beam toward a target location. The first adapter part 100 serves to fix the probe PB so that it does not move.

In this case, the target location may refer to the target point that the end of a needle inserted into a living body needs to reach when the needle of an invasive instrument is inserted into the living body.

The probe PB serves to ultrasonically image the portion of the structure of a blood vessel, into which the needle is to be finally inserted, before the needle is inserted into a living body.

The first adapter part 100 may include a probe fixation portion 110.

The probe PB may be coupled and fixed to the probe fixation portion 110. To this end, the probe fixation portion 110 may be provided with a fixation hole 115 into which the probe PB can be inserted and coupled. The fixation hole 115 may be formed in a shape corresponding to that of the probe PB so that the probe PB does not move in the state of being coupled. The fixation hole 115 may be formed in a shape that penetrates in the vertical direction.

As another example, the probe fixation portion 110 may include a gripper structure composed of a pair of gripper arms. The probe fixation portion 110 including the gripper structure may be in a state in which the pair of gripper arms are open before the probe PB is coupled thereto, and may be changed into a state in which the gripper arms come closer to each other and are closed to fix the probe PB when the probe PB is inserted between the gripper arms.

However, the structures described above are examples. Accordingly, any structure that can fix the location of the probe PB so that the probe PB does not move may be included in embodiments of the probe fixation portion 110 according to the present disclosure.

The first adapter part 100 may further include an adapter body 120.

The adapter body 120 may be connected to the probe fixation portion 110, and may be provided in a form that extends long from the probe fixation portion 110.

In this case, the adapter body 120 may be extended to have a predetermined inclination with respect to the longitudinal axis of the probe PB when the probe PB is coupled to the probe fixation portion 110.

From another point of view, the adapter body 120 may be extended to have a predetermined inclination with respect to the radiation axis PN of the ultrasounds radiated by the probe PB.

The adapter body 120 may have a space formed therein to accommodate the guide part 300 and limitation part 400 to be described later.

As will be described later, the invasive instrument IA is inserted into a living body under the guidance of the guide part 300. The adapter body 120 may also be provided in a form that extends from the probe fixation portion 110 to have a rearward and upward inclination so that the invasive instrument IA can be inserted at a rearward and upward inclination when inserted into a living body.

The second adapter part 200 may be coupled with the invasive instrument IA.

The second adapter part 200 may be provided in a structure that can slide along the guide part 300 to be described later. When the invasive instrument IA is coupled to the second adapter part 200, the second adapter part 200 may be moved together with the invasive instrument IA.

In this case, the invasive instrument IA may be an invasive instrument IV for intravenous injection including a catheter sheath CS and a guide needle GN (see FIG. 1). In this case, the catheter sheath CS refers to a plastic tube that overlaps the needle of the guide needle GN (or that surrounds the needle the outside the needle). The catheter sheath CS may also be referred to as a catheter or catheter tube.

However, the invasive instrument IA described herein is not limited thereto, and any invasive instrument IA that needs to accurately invade up to an intended target location may be included in the invasive instrument IA described herein.

For example, as another embodiment, the invasive instrument IA may include a blood collection device including a blood collection needle IV and a blood container VA. In this case, when blood collection is performed, the blood collection needle IV and the blood container VA move forward together (see FIG. 2(a)). After blood collection has been completed, the blood container VA may be moved rearward and removed (see FIG. 2(b)).

Alternatively, although not shown in the drawings, in another embodiment, the invasive instrument IA may be a biopsy device for obtaining a tissue sample.

The invasive instrument IA may include a needle part configured to invade a living body and a coupling part connected to the needle part. The coupling part may refer to a component that is connected and fixed to an invasive instrument fixation portion 210 to be described later. Based on the above-described embodiments, the needle part may be the catheter sheath CS that overlaps the guide needle GN and is inserted into a living body. Alternatively, the needle part may be the blood collection needle IA. Alternatively, the needle part may be the biopsy needle of the biopsy device.

Based on the above-described embodiments, the coupling part may be the handle of the guide needle GN. Alternatively, the coupling part may be the blood container VA. Alternatively, the coupling part may be the handle of the biopsy device.

Meanwhile, for ease of description, the following description will be made based on the invasive instrument IA shown in FIG. 1 unless otherwise specified.

The second adapter part 200 may include the invasive instrument fixation portion 210.

The invasive instrument IA may be coupled and fixed to the invasive instrument fixation portion 210.

More specifically, the coupling part of the invasive instrument IA may be coupled and fixed to the invasive instrument fixation portion 210.

In an embodiment in which the invasive instrument IA includes a catheter sheath CS and a guide needle GN, the handle of the guide needle GN is coupled and fixed to an invasive instrument fixation portion 210. Accordingly, the overall length of the needle of the guide needle GN may be exposed in front of the invasive instrument fixation portion 210. In the same manner, the overall length of the catheter sheath CS that overlaps the needle of the guide needle GN may also be exposed in front of the invasive instrument fixation portion 210.

In an embodiment in which the invasive instrument IA includes a blood collection needle IV and a blood container VA, the blood container VA may be coupled and fixed to the invasive instrument fixation portion 210. Accordingly, the overall length of the blood collection needle IV may be exposed in front of the invasive instrument fixation portion 210. Through this configuration, there is an effect in that the overall length of even a needle having a short length may be inserted up to a target location, and also there is an effect in that an invasive procedure for a living body may always be performed hygienically.

Meanwhile, the forward direction herein refers to the direction in which the probe fixation portion 110 is arranged. The rearward direction herein refers to the direction in which a rotary knob 420 to be described later is arranged.

The invasive instrument fixation portion 210 may be provided with the fixation hole 215 into which the invasive instrument IA can be inserted and coupled. The fixation hole 215 may be formed in a shape corresponding to that of the invasive instrument IA so that the invasive instrument IA does not move when coupled. The fixation hole 215 may be formed in a shape that penetrates in the front-back direction.

As another example, the invasive instrument fixation portion 210 may include a gripper structure composed of a pair of gripper arms. The invasive instrument fixation portion 210 including the gripper structure may be in a state in which the pair of gripper arms are open before the invasive instrument IA is coupled thereinto, and may be changed into a state in which the gripper arms come closer to each other and are closed to fix the invasive instrument IA when the invasive instrument IA is inserted between the gripper arms.

However, the structures described above are examples. Accordingly, any structure to which the invasive instrument IA can be coupled may be included in embodiments of the invasive instrument fixation portion 210 according to the present disclosure.

The second adapter part 200 may further include a movement portion 220.

One side of the movement portion 220 may be connected to the invasive instrument fixation portion 210. The other side of the movement portion 220 may be connected to the guide part 300 to be described later. In this case, the other side of the movement portion 220 may be connected to the guide part 300 in a form that can slide along the longitudinal direction of the guide part 300.

Based on the state in which the invasive instrument IA is coupled to the invasive instrument fixation portion 210, the longitudinal direction of the guide part 300 may be arranged in parallel with the longitudinal direction of the invasive instrument IA. Accordingly, the movement portion 220 may be slid along the longitudinal direction of the invasive instrument IA.

Based on the state in which the invasive instrument IA is coupled to the invasive instrument fixation portion 210, when the movement portion 220 is slid, the invasive instrument IA may be slid forward or rearward in the state in which the longitudinal axis is fixed.

For example, the forward movement of the invasive instrument IA may insert the catheter sheath CS into a living body. For example, the rearward movement of the invasive instrument IA may eject the guide needle GN out of the living body.

For example, the forward movement of the invasive instrument IA may insert the blood collection needle IV into a living body. For example, the rearward movement of the invasive instrument IA may eject the blood container VA out of the living body.

The guide part 300 may be connected to the second adapter part 200.

The guide part 300 may serve to guide the second adapter part 200 through its movement along the longitudinal direction of the invasive instrument IA.

For example, the guide part 300 may be configured as a rail member that has an open bottom and stop protrusions formed at the bottom ends of both side walls. In this case, the upper end of the movement portion 220 may be formed in a shape corresponding to that of the rail member so that it can be fitted into the rail member.

Through this structure, the second adapter part 200 may be slid along the longitudinal direction of the guide part 300. From another point of view, the second adapter part 200 may be slid along the longitudinal direction of the invasive instrument IA that is coupled thereto.

However, the above-described structure is an example. Accordingly, the movement portion 220 that can slide the second adapter part 200 may be included in embodiments of the guide part 300 according to the present disclosure.

The guide part 300 may be accommodated in the adapter body 120. The guide part 300 may be extended long along the longitudinal direction of the adapter body 120. That is, the guide part 300 may be extended long in the front-rear direction.

The limitation part 400 may be connected to the guide part 300.

The limitation part 400 serves to limit the movement distance of the second adapter part 200. To this end, at least a portion of the limitation part 400 may be placed on the movement path of the second adapter part 200 and limit the movement of the second adapter part 200.

More specifically, the limitation part 400 may include a limitation part body 410. The limitation part body 410 may be formed in a cylindrical shape. The limitation part body 410 may be accommodated in a space inside the adapter body 120. The limitation part body 410 may be arranged in parallel with the guide part 300, and may be arranged above the guide part 300.

A spiral stopper movement groove 411a may be formed along the outer circumferential surface of the limitation part body 410. A stopper 430 to be described later may be fitted into the stopper movement groove 411a.

The limitation part 400 may further include the rotary knob 420.

The rotary knob 420 may be connected to one side of the limitation part body 410 in the longitudinal direction. The rotary knob 420 may be exposed out of the adapter body 120. Through this configuration, a user may hold and rotate the rotary knob 420.

The rotary knob 420 may be formed in a cylindrical shape that is coaxial with the limitation part body 410. Accordingly, when the rotary knob 420 is rotated, the limitation part body 410 connected to the rotary knob 420 may also be rotated together with the rotary knob 420.

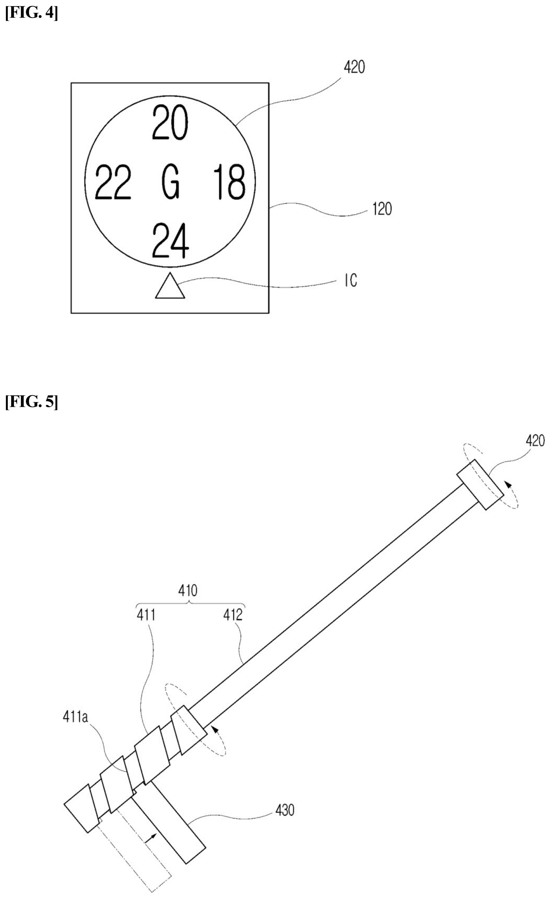

FIG. 4 is a diagram showing the rotary knob of an invasive instrument guide device according to one embodiment of the present disclosure.

Referring to FIG. 4, a plurality of numbers indicating the specifications of the needle included in the invasive instrument IA may be marked on the rotary knob 420. For example, numbers indicating the gauges G of the needle may be marked along the outer circumferential surface of the rotary knob 420. In this case, as the number of each of the gauges increases, the thickness of the needle becomes thicker and the length of the needle becomes smaller. In another embodiment, numbers indicating the lengths of the needle may be marked on the rotary knob 420.

A pointer IC may be marked on one side of the adapter body 120 in which the rotary knob 420 is arranged. A number pointed to by the pointer IC is the specification of a selected needle, and a user may rotate the rotary knob 420 so that the pointer IC may point to a needle having a desired specification.

Referring to FIG. 3 again, the limitation part 400 may further include the stopper 430.

The stopper 430 may be connected to one side of the limitation part body 410 in the radial direction. The stopper 430 may be arranged on the movement path of the second adapter part 200. The stopper 430 may be coupled to the limitation part body 410, and may be coupled to protrude out of the limitation part body 410 in the radial direction. The stopper 430 may protrude toward the guide part 300.

One side of the stopper 430 may be fitted into the stopper movement groove 411a, and the other side thereof may be connected to the guide part 300.

More specifically, one side of the stopper 430 may be movably fitted into the stopper movement groove 411a. The other side of the stopper 430 may be coupled to the guide part 300 in a form that surrounds the guide part 300.

In this case, the other side of the stopper 430 may be configured to have a structure that can slide along the guide part 300 like the movement part 220 described above. In an embodiment in which a guide part 300 includes a rail member, some components arranged on the other side of the stopper 430 may be configured in a form that can be fitted into the rail member.

Through this configuration, when the limitation part body 410 is rotated, one side of the stopper 430 is moved along the stopper movement groove 411a, and accordingly, the stopper 430 may be moved forward or rearward along the longitudinal direction of the guide part 300. From another point of view, the stopper 430 may be moved forward or rearward along the longitudinal direction of the invasive instrument.

FIG. 5 is a diagram illustrating the movement of a stopper in an invasive instrument guide device according to one embodiment of the present disclosure.

The rotary knob 420 may be rotated by a user. As described above, a user may rotate the rotary knob 420 so that the pointer IC can point to the specification of a needle desired to be inserted into a living body.

The limitation part body 410 may be rotated in conjunction with the rotary knob 420. When the limitation part body 410 is rotated, the stopper 430 may be moved along the stopper movement groove 411a that spirally surrounds the outer surface of the limitation part body 410. That is, the stopper 430 may be moved forward or rearward along the longitudinal direction of the invasive instrument IA by the rotation of the rotary knob 420.

In this case, the movement along the longitudinal direction of the invasive instrument IA means that the limitation part body 410, the guide part 300, and the invasive instrument IA are arranged in parallel with each other based on the state in which the invasive instrument IA is coupled with the second adapter part 200. Accordingly, from another point of view, the stopper 430 may be moved along the longitudinal direction of the limitation part body 410. From still another point of view, the stopper 430 may be moved along the longitudinal direction of the guide part 300.

Meanwhile, the limitation part body 410 may include a first body 411 configured such that the stopper movement groove 411a is formed therein, and a second body 412 connected between the rotary knob 420 and the first body 411. The second body 412 may be configured in a cylindrical shape having a smaller diameter than that of the first body 411.

Referring back to FIG. 3, the stopper 430 may be arranged on the movement path of the second adapter part 200 and limit the movement of the second adapter part 200. More specifically, the stopper 430 may be arranged to face the movement part 220 and limit the movement so that the movement part 220 may no longer move forward along the longitudinal direction of the invasive instrument IA.

As described with reference to FIG. 5, the location of the stopper 430 may be varied. Accordingly, the movable distance of the second adapter part 200 limited by the stopper 430 may also be varied.

More specifically, when the stopper 430 is positioned close to the front end of the limitation part body 410, the movable distance of the second adapter part 200 becomes relatively long. When the stopper 430 is positioned close to the rear end of the limitation part body 410, the movable distance of the second adapter part 200 becomes relatively short.

In this case, the location of the stopper 430 according to the rotation angle of the rotary knob 420 may be appropriately set in advance so that the catheter sheath CS can be inserted up to an exact target point.

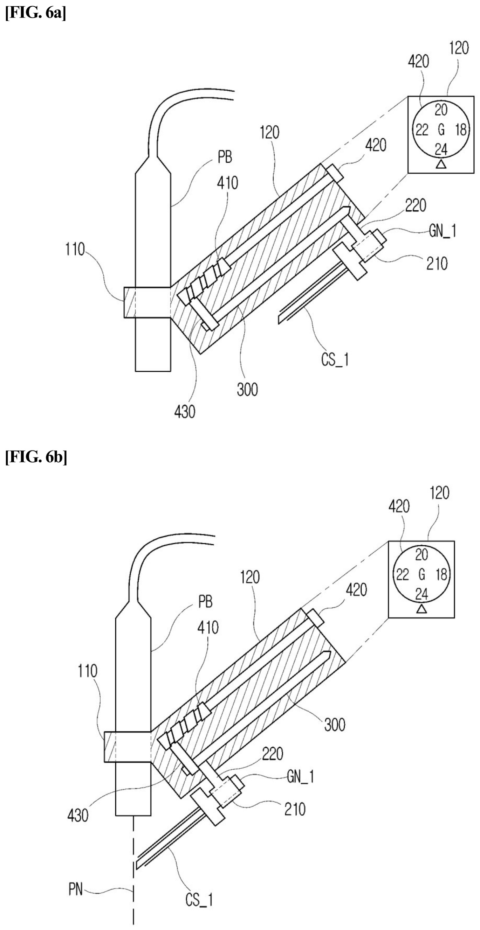

FIG. 6 shows diagrams illustrating an example in which an invasive instrument having a first specification is guided through its insertion by an invasive instrument guide device according to one embodiment of the present disclosure.

Referring to FIG. 6, an invasive instrument IA_1 having a first specification may correspond to a case where a needle is 24G. The invasive instrument IA_1 having a first specification may include a guide needle GN_1 and a catheter sheath CS_1.

The 24G needle is a relatively thin and short needle.

First, a guide needle GN_1 is inserted and fixed into an invasive instrument fixation portion 210. The catheter sheath CS_1 is exposed forward out of the invasive instrument fixation portion 210. The invasive instrument IA_1 and a guide part 300 may be arranged in parallel with each other.

A user may rotate the rotary knob 420 so that the pointer IC points to 24. The location of the stopper 430 may be determined by the rotation of the rotary knob 420. In this case, the length of the needle is short, so that the movement distance of the second adapter part 200 needs to be relatively long in order for the catheter sheath CS_1 to be inserted up to the same target point. That is, the stopper 430 may be arranged close to the front end of the limitation part body 410 (see FIG. 6(a)).

When a user pushes and moves the second adapter part 200 forward, the second adapter part 200 is slid along the guide part 300. Accordingly, the invasive instrument IA_1 may be moved forward in the state of maintaining its longitudinal axis. That is, the invasive instrument IA_1 may be inserted into a living body in the state in which the angle formed by the movement path and the radiation axis PN of ultrasounds is fixed.

As a result, there is an effect in that the invasive instrument IA_1 is guided through its movement path so that the needle is accurately inserted into a target location.

The second adapter part 200 moving together with the invasive instrument IA_1 meets the stopper 430, and stops further forward movement. At the point where the movement of the second adapter part 200 is limited by the stopper 430, the end of the catheter sheath CS_1 is inserted exactly up to a target point (see FIG. 6(b)).

Thereafter, the user may move the guide needle GN_1 rearward while leaving the catheter sheath CS_1 behind by pulling the second adapter part 200, thereby separating the catheter sheath CS_1 and the guide needle GN_1 from each other.

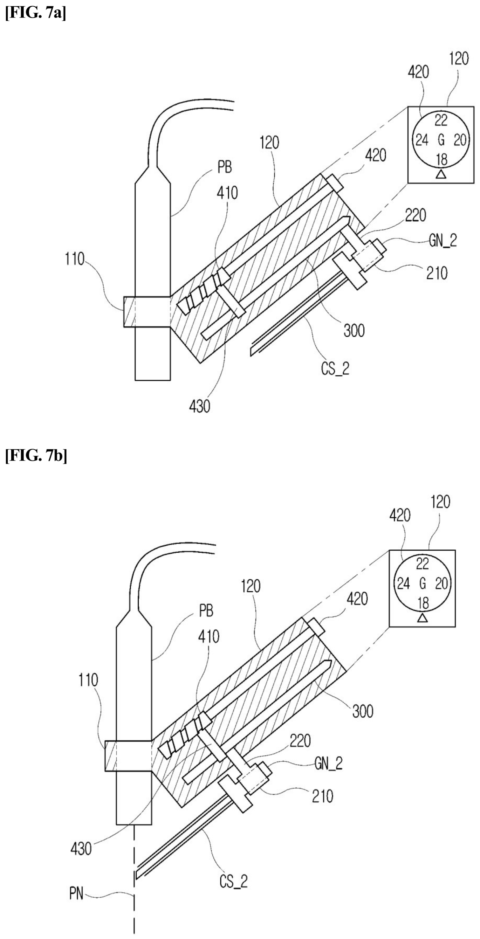

FIG. 7 shows diagrams illustrating an example in which an invasive instrument having a second specification is guided through its insertion by an invasive instrument guide device according to one embodiment of the present disclosure.

Referring to FIG. 7, an invasive instrument IA_2 having a second specification may correspond to a case where a needle is 18G. The invasive instrument IA_2 having a second specification may include a guide needle GN_2 and a catheter sheath CS_2.

The 18G needle is a relatively thick and long needle.

First, the guide needle GN_2 is inserted and fixed into an invasive instrument fixation portion 210. The catheter sheath CS_2 is exposed forward out of the invasive instrument fixation portion 210. The invasive instrument IA_2 and a guide part 300 may be arranged in parallel with each other.

The user may rotate a rotary knob 420 so that a pointer IC points to 18. The location of the stopper 430 may be determined by the rotation of the rotary knob 420. In this case, the length of the needle is long, so that the movement distance of a second adapter part 200 needs to be relatively short in order for the catheter sheath CS_2 to be inserted up to the same target point. That is, the stopper 430 may be arranged close to the rear end of a limitation part body 410 (see FIG. 7(a)).

When the user pushes and moves the second adapter part 200 forward, the second adapter part 200 is slid along the guide part 300. Accordingly, the invasive instrument IA_2 may be moved forward in the state of maintaining its longitudinal axis. That is, the invasive instrument IA_2 may be inserted into a living body in the state in which the angle formed between the movement path and the radiation axis PN of ultrasounds is fixed.

As a result, there is an effect in that the invasive instrument IA_2 is guided through its movement path so that a needle is accurately inserted up to a target location.

The second adapter part 200 moving together with the invasive instrument IA_2 meets the stopper 430, and stops further forward movement. At the point where the movement of the second adapter part 200 is limited by the stopper 430, the end of the catheter sheath CS_2 is inserted exactly up to a target point (see FIG. 7(b)).

Thereafter, the user may move the guide needle GN rearward while leaving the catheter sheath CS_2 behind by pulling the second adapter part 200, thereby separating the catheter sheath CS_2 and the guide needle GN_2 from each other.

So far, there has been described the embodiment in which the stopper 420 is moved along the stopper movement groove 411a formed on the outer circumferential surface of the limitation part body 410.

The present invention is not limited thereto. As another embodiment, the stopper 420 may be moved along a linear rail formed in the limitation part body 410. In this case, the shape of the limitation part body 410 is not limited to a cylindrical shape. As still another embodiment, a driving motor configured to move the stopper 420 may be provided, and the stopper 420 may be moved using the rotational force of the driving motor.

In addition, an embodiment that can vary the location of the stopper 420 in order to accurately invade the invasive instrument IA into a target location may be included in embodiments of the present invention.

As described above, the invasive instrument guide device according to one embodiment of the present disclosure includes the second adapter part configured such that the invasive instrument is coupled thereto and the guide part configured to guide the second adapter part through its sliding along the longitudinal direction of the invasive instrument. Accordingly, there is an effect in that a needle may be accurately inserted up to a target location.

Furthermore, the invasive instrument guide device according to one embodiment of the present disclosure is configured such that the movable distance of the second adapter part is variable. Accordingly, there is an effect in that even when the length of the needle is changed, the needle may always be accurately inserted up to the same target location.

Furthermore, the invasive instrument guide device according to one embodiment of the present disclosure is configured such that the coupling part of the invasive instrument is fixed to the second adapter part and the needle part that directly invades a living body is exposed out of the invasive instrument guide device. Accordingly, there is an effect in that a portion of the needle is not covered by the invasive instrument guide device, so that the overall length of even a needle having a short length can be inserted up to a target position.

Moreover, the invasive instrument guide device according to one embodiment of the present disclosure is configured such that the coupling part of the invasive instrument is fixed to the second adapter part and the needle part that directly invades a living body is exposed out of the invasive instrument guide device. Accordingly, there is an effect in that the needle does not come into contact with one component of the invasive instrument guide device, so that an invasive procedure for a living body may always be performed hygienically.

The various embodiments of the present disclosure described above may be combined with one or more additional embodiments, and may be changed within the range understandable to those skilled in the art in light of the above detailed description. The embodiments of the present disclosure should be understood as illustrative but not restrictive in all respects. For example, individual components described as unitary may be implemented in a distributed manner, and similarly, the components described as distributed may also be implemented in a combined form. Accordingly, all changes or modifications derived from the meanings and scopes of the claims of the present disclosure and their equivalents should be construed as being included in the scope of the present disclosure.

## Claims

1. An invasive instrument guide device comprising:
a first adapter part configured to be coupled with a probe that radiates an ultrasound beam toward a target location, and to fix a location of the probe;
a second adapter part configured to be coupled with an invasive instrument, and to be moved together with the invasive instrument;
a guide part configured to be connected to the second adapter part, and to guide the second adapter part through its movement along a longitudinal direction of the invasive instrument; and
a limitation part including a stopper configured to be arranged on a movement path of the second adapter part and limit a movable distance of the second adapter part;
wherein the limitation part is controlled such that the movable distance of the second adapter part is changed.

2. The invasive instrument guide device of claim 1, wherein the first adapter part comprises:
a probe fixation portion configured such that the probe is coupled and fixed thereto; and
an adapter body configured to be connected to the probe fixation portion, and to accommodate the guide part and the limitation part in an internal space thereof.

3. The invasive instrument guide device of claim 1, wherein the second adapter part comprises:
an invasive instrument fixation portion configured such that the invasive instrument is coupled and fixed thereto; and
a movement portion configured such that one side thereof is connected to the invasive instrument fixation portion and a remaining side thereof is connected to the guide part, and such that it is slid along the longitudinal direction.

4. The invasive instrument guide device of claim 3, wherein the invasive instrument comprises:
a needle part configured to invade a living body; and
a coupling part configured to be connected to the needle part, and to be coupled to the invasive instrument fixation portion.

5. The invasive instrument guide device of claim 1, wherein the limitation part further comprises:
a limitation part body; and
a rotary knob configured to be connected to one side of the limitation part body in a longitudinal direction thereof, and to be rotated by an external force;
wherein the stopper is connected to one side of the limitation part body in a radial direction thereof, and is arranged on a movement path of the second adapter part.

6. The invasive instrument guide device of claim 5, wherein the limitation part body has a cylindrical shape, and is provided with a spiral stopper movement groove formed along an outer circumferential surface thereof.

7. The invasive instrument guide device of claim 6, wherein the stopper is configured such that one side thereof is connected to the stopper movement groove and a remaining side thereof is connected to the guide part.

8. The invasive instrument guide device of claim 5, wherein a plurality of numbers each indicating a gauge specification or length of a needle included in the invasive instrument are marked on the rotary knob.

9. The invasive instrument guide device of claim 5, wherein the limitation part body is rotated in conjunction with the rotary knob when the rotary knob is rotated.

10. The invasive instrument guide device of claim 5, wherein the stopper is moved forward or rearward along the longitudinal direction when the rotary knob is rotated.
